(19) Europäisches Patentamt
European Patent Office
Office européen des brevets

(11) Publication number: 0 007 142 B1

(12) # EUROPEAN PATENT SPECIFICATION

(45) Date of publication of patent specification: 12.05.82

(21) Application number: 79200379.0

(22) Date of filing: 09.07.79

(51) Int. Cl.³: C 07 C 43/172, C 07 C 61/16, C 07 C 69/145, C 07 C 69/743

(54) **Novel intermediates in the preparation of cyclopropanecarboxylate esters and process for their manufacture.**

(30) Priority: 19.07.78 GB 3033978
19.07.78 GB 3034078
17.08.78 GB 3376378

(43) Date of publication of application: 23.01.80 Bulletin 80/2

(45) Publication of the grant of the patent: 12.05.82 Bulletin 82/19

(84) Designated Contracting States: BE CH DE FR GB IT NL

(56) References cited:
Chemical Abstracts, Volume 75, Nr. 13 1971 (COLUMBUS, OHIO, USA)
G. LAVIELLE et al.: "Anions associated with the chlorotris(dimethyl)phosphonium cation. II. Synthesis of aliphatic 1,1-dichloro olefins. Reaction mechanism."
page 317, column 1, abstract Nr. 88029 Y

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Van Berkel, Johannes
Badhuisweg 3
NL-1031 CM Amsterdam (NL)
Inventor: Kelderman, Hendrik Cornelis
Badhuisweg 3
NL-1031 CM Amsterdam (NL)
Inventor: Verbrugge, Peter Adriaan
Badhuisweg 3
NL-1031 CM Amsterdam (NL)
Inventor: Kramer, Petrus Anthonius
Badhuisweg 3
NL-1031 CM Amsterdam (NL)
Inventor: Syrier, Johannes Leopold Marie
Badhuisweg 3
NL-1031 CM Amsterdam (NL)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Novel intermediates in the preparation of cyclopropanecarboxylate esters and process for their manufacture

The invention relates to compounds which are useful intermediates in the preparation of stereo-isomeric cyclopropanecarboxylate esters. The invention also relates to a process for the preparation of these intermediates.

The stereo-isomeric cyclopropanecarboxylate esters are insecticidally-active compounds known as "pyrethroids" and as they combine exceptionally good insecticidal properties with a very low mammalian toxicity, they are of considerable interest to the agrochemical industry and much effort has been expended in finding economic routes to them and to their principal intermediates.

The general formula of one class of these pyrethroid compounds may be represented as follows:

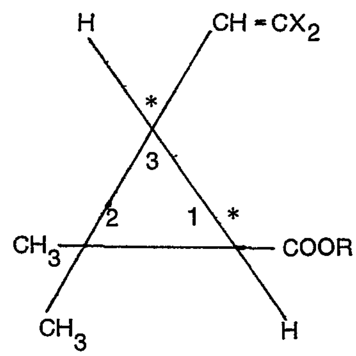

(I)

where each asterisk denotes an asymmetric carbon atom; each X is a halogen atom; and R is a member of a group of radicals known to impart insecticidal activity to the molecule, e.g., 3-phenoxybenzyl or *alpha*-cyano-3-phenoxybenzyl. It is known that the stereo-isomeric form of the acid portion of the ester of formula I should be in the (1R,*cis*) form for maximum insecticidal activity, i.e., the absolute configuration at carbon atom 1 is R and the two hydrogen atoms on carbon atoms 1 and 3 are in a *cis* relationship. This nomenclature is known as the Elliott nomenclature and is defined in M. Elliott, A.W. Farnham, N.F. James, P.H. Needham and D.A. Pullman, Nature, 1974, *248*, 710.

It follows, therefore, that if these stereo-isomeric esters of formula I are to be prepared, either a stereo-specific chemical route is required or the desired stereo-isomer must be obtained from a racemic form by physical separation techniques. The latter are expensive and laborious and not readily employed on an industrial scale. The Applicant has found a stereo-specific route which uses as starting material the naturally-occurring substance (+)-3-carene whose formula is as follows:

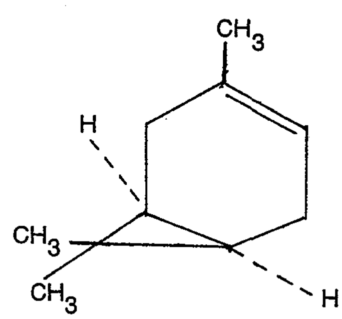

(II)

This compound is an inexpensive readily-available natural terpene and the present application relates to intermediates in a route to the (1R,*cis*)-acid portion of the pyrethroid ester of formula I starting from (+)-3-carene.

The present invention provides compounds of the general formula:

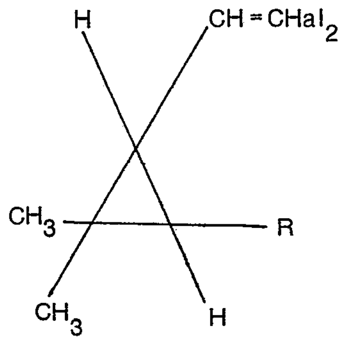

(III)

wherein each Hal represents a halogen atom, especially chlorine or bromine, and R a group selected from:

$-CH_2-CO-O-R^1$ (i)

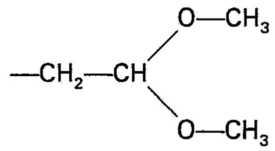

(ii)

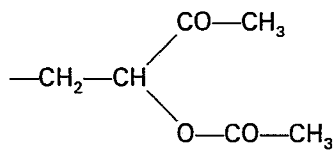

(iii)

wherein $R^1$ is a hydrogen atom or an alkyl group, preferably containing 1 to 4 carbon atoms, e.g., a methyl group, or a salt-forming cation, e.g., alkali metal, ammonium or alkyl-substituted ammonium, the compounds being in the same stereo-isomeric form as that of the cyclopropane ring of (+)-3-carene.

The three groups which R may represent in formula III may be named as follows:

(i) alkoxycarbonylmethyl or carboxymethyl and salts thereof,
(ii) . 2,2-dimethoxyethyl, and
(iii) 2-methylcarbonyloxy-3-oxobutyl.

Preferred compounds of the general formula III are:

methyl 2-(2,2-dichlorovinyl)-3,3-dimethylcyclopropylacetate;
2-[2-(2,2-dichlorovinyl)-3,3-dimethylcyclopropyl]ethanal dimethyl acetal; and
2-[2-(2,2-dichlorovinyl)-3,3-dimethylcyclopropyl]-1-acetylethyl acetate;

the compounds having the same stereo-isomeric form as that of the cyclopropane ring of (+)-3-carene, e.g., the compounds prepared in the Examples.

The compounds according to the invention may be prepared by processes known per se, for example, according to the methods disclosed in U.K. Patent Specification 1,413,491 which discloses the preparation of dihalovinylcyclopropyl compounds by reacting 3-formyl-2,2-dimethylcyclopropane carboxylate (which for the purposes of the invention should be in the same stereo-isomeric form as that of the cyclopropane ring of (+)-3-carene) with a dihalomethyhlenephosphorane (which can be prepared by reaction of a tri-organophosphine, normally triphenylphosphine, with a carbon tetrahalide).

The compounds according to the invention are preferably prepared by a process characterized in that (a) a tri(dialkylamino)phosphine or an alkyl ester of an orthophosphorous acid bis(dialkylamide) is reacted with a tetrahalomethane or a trihalomethane and (b) the product resulting from the first step is reacted with an aldehyde of the general formula:

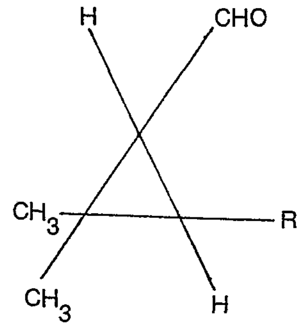

(IV)

wherein R has the same meaning as in the general formula III, and the aldehyde is in the same stereoisomeric form as that of the cyclopropane ring of (+)-3-carene, both steps being carried out in the presence of a substantially inert solvent. Preferably step (a) is allowed to proceed to substantial completion.

The alkyl groups present in the tri(dialkylamino)phosphine or the alkyl ester of an orthophosphorous acid bis(dialkylamide) may be the same or different and linear or branched. The alkyl groups are suitably the same, have preferably less than six carbon atoms and more preferably less than three. The use of tri(dialkylamino)phosphines is preferred, because they usually afford the compounds of formula III in a higher yield than the alkyl esters of ortho-phosphorous acid bis(dialkylamines) )the latter compounds are obtained by replacing one of the dialkylamino groups in a tri(dialkylamino)-phosphine by an alkoxy group). Tri(diethylamino)phosphine and tri(dimethylamino)phosphine are most preferred.

Tri(dialkylamino)phosphines may be prepared by reaction of a dialkylamine with a phosphorous trihalide, as described in "Organic Synthesis", Coll. Vol. V (1973) 602—603. This reaction results in the formation of a solution of the tri(dialkylamino)phosphine which also contains precipitated dialkylammonium halide. According to a feature of the present invention a tri(dialkylamino)phosphine may be prepared by reacting a dialkylamine with a phosphorous trihalide in the presence of a substantially inert solvent. The resulting reaction mixture can then be washed with water to remove unwanted byproducts (whether or not after prior separation of the precipitated dialkylammonium halide) and the tri(dialkylamino)phosphine dissolved in the washed solution reacted with the halomethane. It is not necessary to separate the precipitated dialkylammonium halide prior to washing, because this salt is water-soluble. The yield of the compound of formula III can be further enhanced by drying the washed liquid, for example, over a solid drying agent such as anhydrous sodium sulphate or anhydrous magnesium sulphate.

Another attractive feature of the process according to the present invention is that it may be carried out in the presence of an alkane solvent, for example, alkane solvents with a boiling point or boiling range up to 200°C. This also applies to the said reaction between a dialkylamine and a phosphorous trihalide. Examples of alkane solvents are pentane, hexane, heptane, octane and nonane. Mixtures of alkanes are very suitable, for example, gasolines having a boiling range from 62°C to 82°C or from 80°C to 110°C. If desired, the process may be carried out in substantially inert solvents other than alkanes, for example, in tetrahydrofuran.

Examples of tetrahalomethanes or trihalomethanes which are compounds capable of generating a dihalocarbene under the conditions of the process according to the present invention are carbon tetrahalides, chloroform, bromoform and iodoform. Very good results have been obtained with carbon tetrahalides. Examples of carbon tetrahalides are carbon tetrachloride, carbon tetrabromide, carbon tetra-iodide, bromotrichloromethane (forming dichlorocarbene) and dibromofluoromethane (forming difluorocarbene). Very good results have been obtained with carbon tetrachloride. These halomethanes may also act as solvent of co-solvent for the process according to the invention.

Both steps of the process according to the present invention are preferably carried out at a temperature in the range of from −50°C to +50°C, particularly at temperatures of from −20°C to +35°C.

The compounds and process according to the invention are of interest as part of a multi-step process to pyrethroid insecticides, e.g., esters based on (1R,*cis*)-3-(2,2-dichlorovinyl)-2,2-dimethylcyclopropane carboxylic acid. For example alkyl 2-(2,2-dihalovinyl)-3,3-dimethylcyclopropylacetates available from (+)-3-carene can be converted into the appropriate pyrethroid acid according to the following reaction scheme:

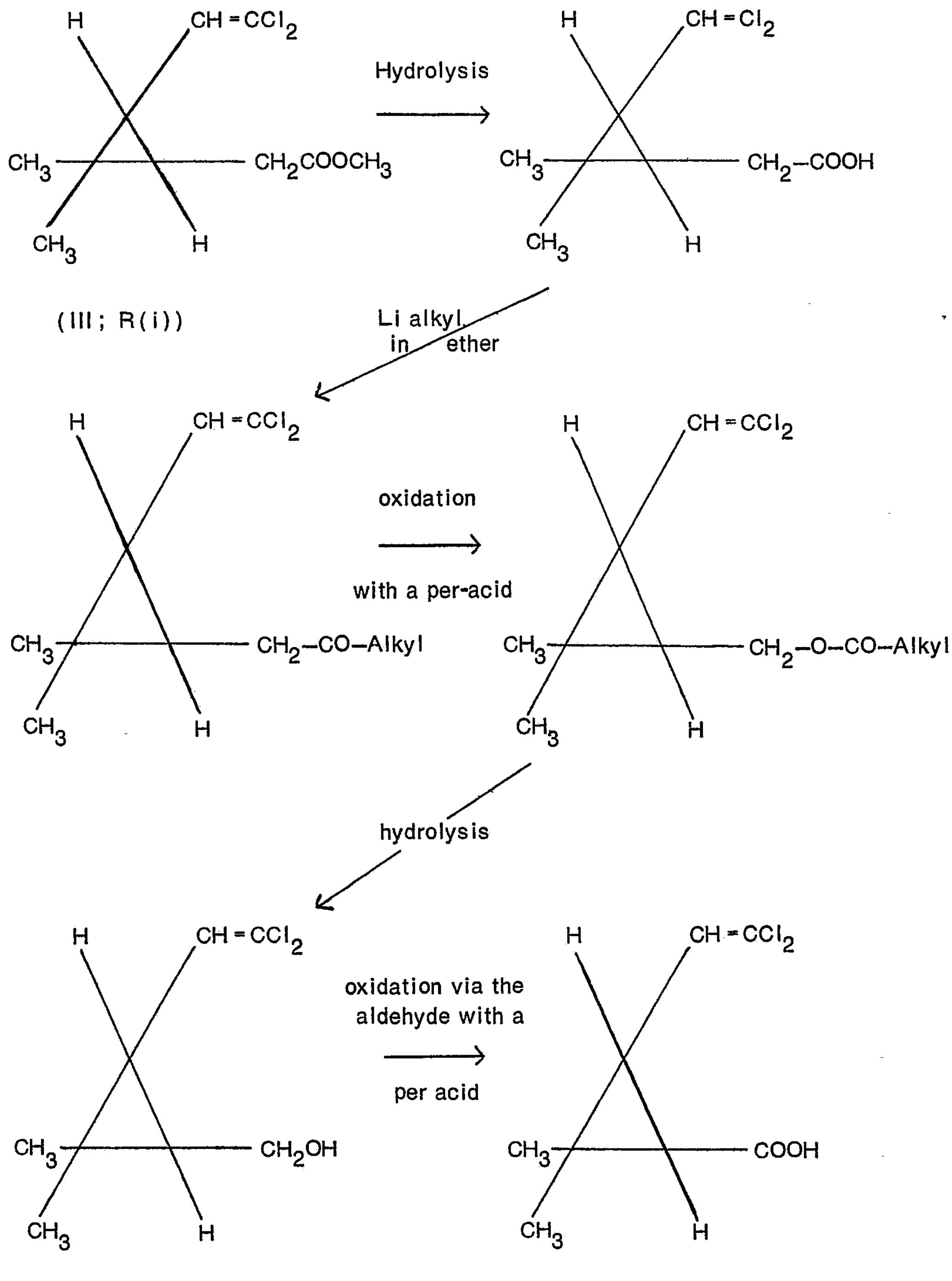


(III; R(i))

Complete Formula T (R=H; Hal=Cl)

This reaction scheme illustrates the manufacture of the pyrethroid (1R,*cis*)-acid (Compound I; R=H Hal=Cl) from one of the novel compounds according to the invention (III; R=i) which itself is readily available from the naturally occurring compound (+)-3-carene. Other reaction schemes can also be drawn for the remaining novel compounds according to the invention. The five intermediate compounds, in racemic, single- or mixture-isomer form, in the above reaction scheme have been prepared and are believed to be novel compounds.

The following Examples further illustrate the invention. Yields and purities were determined by means of gas-liquid chromatography and nuclear magnetic resonance (NMR) spectroscopy. The NMR data quoted were recorded at 90 MHz using solutions of the compounds in deuterochloroform; the absorptions given are relative to a tetramethylsilane standard.

## EXAMPLE I

Preparation of (1R,*cis*)-methyl 2-(2,2-dichlorovinyl)-3,3-dimethylcyclopropylacetate

Tri(dimethylamino)phosphine (6.2 mmol.) was added at once with stirring at a temperature of −10°C to a solution of carbon tetrachloride (5.8 mmol.) in pentane (15 ml), present in a three-necked round-bottomed flask provided with a dropping funnel, magnetic stirrer, thermometer, reflux condenser, calcium chloride tube and in inlet for nitrogen. The temperature was allowed to rise to +10°C, which resulted in the formation of a white precipitate. All of this precipitate was formed at once. This indicated the end of the first step. The suspension was cooled to −10°C, at which temperature (1R,*cis*)-methyl 2-formyl-3,3-dimethylcyclopropylacetate (2.9 mmol.) was added. Then the temperature was allowed to increase to +10°C over a period of 10 min. This ended the second step. The reaction mixture was washed with water (75 ml), the washed liquid was dried over anhydrous sodium sulphate and the solvent was evaporated from the dried liquid, leaving a residue (0.55 g) which contained the title compound (100% 1R,*cis*) in a yield of 81%.

The NMR spectrum of the title compound showed the following absorptions:

| | |
|---|---|
| $\delta$ = 1.03 ppm singlet $H_3C—C—CH_3$ | $\delta$ = 1.19 ppm singlet $H_3C—C—CH_3$ |
| $\delta$ = 1.55 ppm double doublet $HC—CH{=}C$ | $\delta$ = 1.35 ppm multiplet $HC—CH_2$ |
| $\delta$ = 2.33 ppm doublet J=7.5Hz $HC—CH_2$ | $\delta$ = 3.71 ppm singlet $OCH_3$ |
| $\delta$ = 5.53 ppm doublet J=8Hz $CH{=}C$ | |

The optical rotation of a solution of the title compound in methanol was $[\alpha]_D^{20}$ = +32.2°C, concentration 15.39 g/l in methanol.

## EXAMPLE II

Preparation in two steps of (1R,*cis*)-2-[2-(2,2-dichlorovinyl)-3,3-dimethylcyclopropyl]ethanal dimethyl acetal

Tri(dimethylamino)phosphine (168.3 mmol.) was added over a period of 12 minutes to a stirred solution of carbon tetrachloride (167.4 mmol.) in pentane (369 ml) kept at 0°C under nitrogen in a 1 l flask. Then, the mixture in the flask was stirred for 30 minutes at 0°C. This finished the first step.

At 0°C (1R,*cis*)-2-(2,2-dimethoxyethyl)-3,3-dimethylcyclopropanecarbaldehyde (66.4 mmol.) was added dropwise to the suspension in the flask over a period of nine minutes. The temperature was increased to 12°C over a period of 15 minutes and stirring was continued at the temperature for a further 15 minutes. This finished the second step. Then, water (75 ml) was added at 12°C and — after removal of the aqueous phase — the organic phase was washed with two 35-ml portions of water. The washed organic phase was dried over anhydrous magnesium sulphate and the solvent was evaporated from the dried solution to give a residue (17.4 g) containing the title compound (100% (1R,*cis*), purity 88%, yield 91.1%).

The NMR spectrum of the title compound showed the following absorptions:

| | |
|---|---|
| $\delta$ = 1.00 ppm singlet $H_3C—C—CH_3$ | $\delta$ = 1.13 ppm singlet $H_3C—C—CH_3$ |
| $\delta$ = 3.33 ppm singlet $C—(O—CH_3)_2$ | $\delta$ = 4.33 ppm triplet $(H_3C—O)_2—CH—$ |
| $\delta$ = 5.59 ppm doublet $C{=}CH$ | |

multiplets for the two H-atoms bound to the ring and for $HC—CH_2—CH$.

## EXAMPLE III

Preparation in one step of (1R,*cis*)-2-[2-(2,2-dichlorovinyl)-3,3-dimethylcyclopropyl]ethanal dimethyl acetal

Tri(dimethylamino)phosphine (163.8 mmol.) was added with stirring at 0°C to a solution of (1R,*cis*)-2-(2,2-dimethoxyethyl)-3,3-dimethylcyclopropanecarbaldehyde (65.5 mmol.) and carbon tetrachloride (163.2 mmol.) in n-pentane (350 ml) kept under nitrogen in a 1 l flask. After 15 minutes stirring the mixture was warmed up to 14°C and kept at this temperature for 15 minutes. Then, water (75 ml) was added at 14°C and — after removal of the aqueous phase — the organic phase was washed with two 35 ml portions of water. The washed organic phase was dried over anhydrous magnesium sulphate and the solvent was evaporated from the dried solution to give a residue (15.7 g) containing 32.1 mmol. of the title compound (100% (1R,*cis*), purity 51.7%, yield 49%). Comparison with the yield of the title compound obtained in Example II shows that the two-step process for the preparation of the title compound gives considerably higher yields.

## EXAMPLE IV

Preparation in two steps of (1R,*cis*)-2-[2-(2,2-dichlorovinyl)-3,3-dimethylcyclopropyl]-1-acetylethyl acetate

Tri(dimethylamino)phosphine (40 mmol.) was added at −20°C and with stirring under nitrogen to a 250 ml flask charged with carbon tetrachloride (40 mmol.) and diethyl ether (160 ml). Then, the temperature was allowed to rise to +10°C. This finished the first step. The resulting suspension was

cooled to −20°C and a solution in diethyl ether (5 ml) of the 1R, as isomer of 2-(2-formyl-3,3-dimethylcyclopropyl)-1-acetylethyl acetate was added. Then, the temperature of the mixture was allowed to rise to 20°C. This finished the second step. Water (50 ml) was added, the mixture was stirred for 5 minutes and, after settling, the organic phase was isolated and washed with two 50 ml portions of water. The washed organic liquid was dried over anhydrous magnesium sulphate and the solvent was evaporated from the dried liquid at 1.3 kPa to leave a residue (3.5 g) containing the title compound (100% (1R,*cis*), yield 84%).

The NMR spectrum of the title compound showed the following absorptions:

$\delta = 1.04$ ppm singlet $H_3C$—C—$CH_3$ $\delta = 1.16$ ppm singlet $H_3C$—C—$CH_3$
$\delta = 2.19$ ppm singlet $H_3C$—C(O)—C and $\delta = 5.59$ ppm doublet $HC{=}CCl_2$
$H_3C$—C(O)—O—
$\delta = 5.05$ ppm doublet of doublets $H_2C$—$CH$—O—

multiplets for each of the H-atoms bound to the ring and for HC—$CH_2$—CH.

EXAMPLE V

Preparation in one step of (1R,*cis*)-2-[2-(2,2-dichlorovinyl)-3,3-dimethylcyclopropyl]-1-acetylethyl acetate

Tri(dimethylamino)phosphine (40 mmol.) was added with stirring at −20°C to a solution of 2-(2-formyl)-3,3-dimethylcyclopropyl-1-acetylethyl acetate (14.2 mmol.) and carbon tetrachloride (40 mmol.) in diethyl ether (160 ml) kept under nitrogen. Then, the temperature of the mixture was allowed to rise to 20°C. Water (50 ml) was added, the mixture was stirred for 5 minutes and, after settling, the organic phase was isolated. The organic phase was washed with two 50 ml portions of water. The washed organic phase was dried over anhydrous magnesium sulphate and the solvent was evaporated from the dried solution to leave a residue (2.2 g) containing the title compound (100% (1R,*cis*), yield 54%).

**Claims**

1. Compounds of the general formula:—

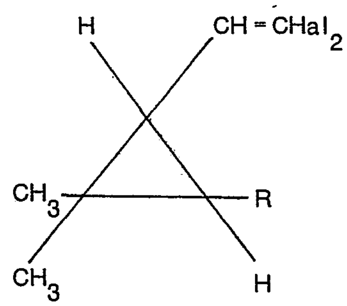

(III)

wherein each Hal is halogen atom and R is a group selected from:

—$CH_2$—CO—O—$R^1$ (i)

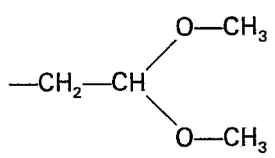

and (ii)

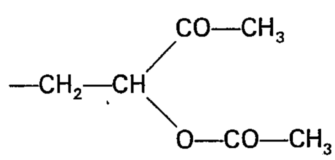

(iii)

wherein $R^1$ is a hydrogen atom or an alkyl group, or a salt-forming cation, the compounds being in the same stereo-isomeric form as that of the cyclopropane ring of (+)-3-carene.

2. Compounds according to claim 1, characterized in that $R^1$ is an alkyl group containing 1 to 4 carbon atoms, or an alkali metal or ammonium or alkyl-substituted ammonium cation.

3. A process for the preparation of compounds of formula III characterized in that (a) a tri(dialkylamino)phosphine or an alkyl ester of an ortho-phosphorous acid bis(dialkylamide) is reacted with a tetrahalomethane or a trihalomethane and (b) the product resulting from the first step is reacted with an aldehyde of the general formula:—

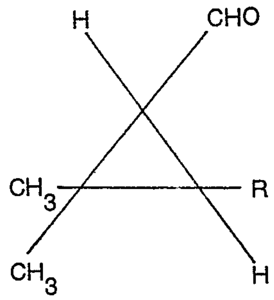

(IV)

where R has the same meaning as in the general formula III and the aldehyde is in the same stereoisomeric form as that of the cyclopropane ring of (+)-3-carene, both steps being carried out in the presence of a substantially inert solvent.

4. A process according to claim 3, characterized in that the tri(dialkylamino)phosphine is tri(diethylamino)phosphine or tri(dimethylamino)phosphine.

5. A process according to claim 3 or 4, characterized in that the substantially inert solvent is an alkane or tetrahydrofuran.

6. A process according to any one of claims 3 to 5, characterized in that the tetrahalo- or trihalomethane is a carbon tetrahalide, chloroform, bromoform or iodoform.

7. A process according to claim 3 or 4, characterized in that the tetrahalomethane is employed as the substantially inert solvent.

8. A process according to any one of claims 3 to 7, characterized in that the process is carried out at a temperature in the range −50°C to +50°C.

**Revendications**

1. Composés de la formule générale:

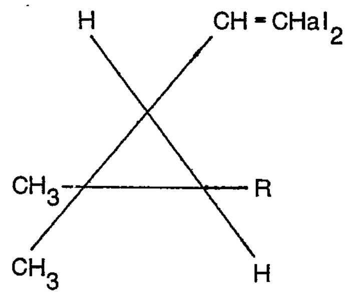

(III)

dans laquelle chaque Hal est un atome d'halogène et R est un groupe choisi parmi les suivants:

$-CH_2-CO-O-R^1$ (i)

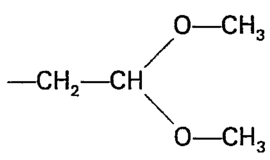

et (ii)

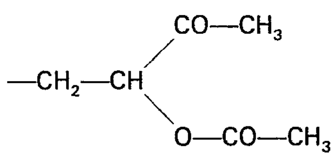

(iii)

où $R^1$ est un atome d'hydrogène ou un groupe alcoyle, ou un cation formant des sels, les composés étant dans la même forme stéréo-isomère que celle du noyau de cyclopropane du (+)-3-carène.

2. Composés selon la revendication 1, caractérisés en ce que $R^1$ est un groupe alcoyle contenant de 1 à 4 atomes de carbone ou un cation de métal alcalin ou d'ammonium ou d'ammonium alcoylé.

3. Procédé pour la préparation de composés de formule III, caractérisé en ce que (a) on fait réagir une tri(dialcoylamino)phosphine ou un ester d'alcoyle d'un bis-(dialcoylamide) d'acide orthophosphoreux avec un tétrahalométhane ou un trihalométhane et (b) le produit résultant de la première étape est mis à réagir avec un aldéhyde de la formule générale:

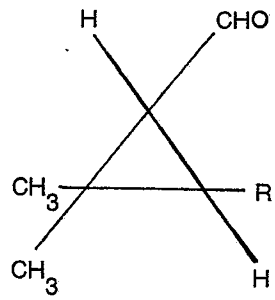

(IV)

dans laquelle R a la même signification que dans la formule générale III et l'aldéhyde est dans la même forme stéréo-isomère que celle du noyau de cyclopropane du (+)-3-carène, les deux étapes étant conduites en présence d'un solvant sensiblement inerte.

4. Procédé selon la revendication 3, caractérisé en ce que la tri(dialcoylamino)phosphine est la tri-(diéthylamino)phosphine ou la tri(diméthylamino)phosphine.

5. Procédé selon l'une des revendications 3 et 4, caractérisé en ce que le solvant sensiblement inerte est un alcane ou du tétrahydrofuranne.

6. Procédé selon l'une quelconque des revendications 3 à 5, caractérisé en ce que le tétrahalo- ou trihalométhane est un tétrahalogénure de carbone, le chloroforme, le bromoforme ou l'iodoforme.

7. Procédé selon l'une des revendications 3 et 4, caractérisé en ce que le tétrahalométhane est utilisé en tant que solvant sensiblement inerte.

8. Procédé selon l'une quelconque des revendications 3 à 7, caractérisé en ce que le procédé est mis en oeuvre à une température comprise entre −50°C et +50°C.

**Patentansprüche**

1. Verbindungen der allgemeinen Formel

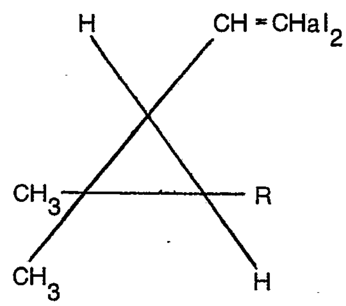

(III)

in der jedes Hal ein Halogenatom und R eine Gruppe ausgewählt aus

$$-CH_2-CO-O-R^1 \quad (i)$$

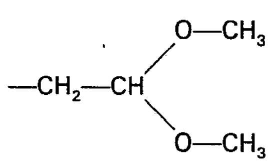

und

(ii)

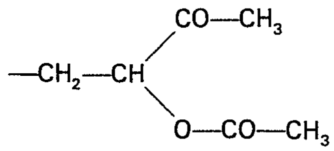

(iii)

ist, wobei $R^1$ ein Wasserstoffatom oder eine Alkylgruppe oder ein salzbildendes Kation bedeutet und die Verbindungen in der gleichen stereoisomeren Form vorliegen wie der Cyclopropanring von (+)-3-Caren.

2. Verbindungen nach Anspruch 1, dadurch gekennzeichnet, dass $R^1$ eine Alkylgruppe enthaltend 1 bis 4 Kohlenstoffatome oder ein Alkalimetall-, oder Ammonium- oder Alkyl-substituiertes Ammoniumkation ist.

3. Verfahren zur Herstellung der Verbindungen der Formel III, dadurch gekennzeichnet, dass (a) ein Tri(dialkylamino)phosphin oder ein Alkylester eines ortho-Phosphorsäure-bis(dialkylamid) umgesetzt wird mit einem Tetrahalogenmethan oder einem Trihalogenmethan und (b) das aus der ersten Stufe resultierende Produkt umgesetzt wird mit einem Aldehyd der allgemeinen Formel

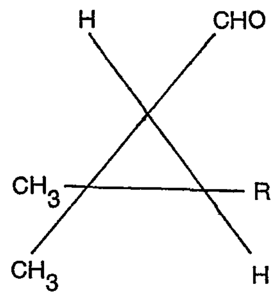

(IV)

in der R die gleiche Bedeutung wie in der allgemeinen Formel III hat und der Aldehyd die gleiche stereoisomere Form aufweist wie der Cyclopropanring von (+)-3-Caren und wobei beide Stufen in Gegenwart eines praktisch inerten Lösungsmittels ausgeführt werden.

4. Verfahren nach Anspruch 3, dadurch gekennzeichnet, dass das Tri(dialkylamino)phosphin Tri(diäthylamino)phosphin oder Tri(dimethylamino)phosphin ist.

5. Verfahren nach Anspruch 3 oder 4, dadurch gekennzeichnet, dass das praktisch inerte Lösungsmittel ein Alkan oder Tetrahydrofuran ist.

6. Verfahren nach einem der Ansprüche 3 bis 5, dadurch gekennzeichnet, dass das Tetrahalogen- oder Trihalogenmethan ein Tetrahalogenkohlenstoff, Chloroform, Bromoform oder Jodoform ist.

7. Verfahren nach Anspruch 3 oder 4, dadurch gekennzeichnet, dass das Tetrahalogenmethan als das praktisch inerte Lösungsmittel verwendet wird.

8. Verfahren nach einem der Ansprüche 3 bis 7, dadurch gekennzeichnet, dass das Verfahren bei einer Temperatur im Bereich von –50°C bis +50°C ausgeführt wird.